# FASCICULE DE BREVET EUROPEEN

(11) **EP 1 405 653 B1**
(45) Date de publication et mention de la délivrance du brevet: **03.05.2006**
(21) Numéro de dépôt: 03292452.4
(22) Date de dépôt: 03.10.2003
(51) Int. Cl.: A61N 1/362, A61N 1/365

(54) **Dispositif médical implantable actif, notamment stimulateur cardiaque, défibrillateur, cardioverteur et/ou dispositif multisite, à discrimination des extrasystoles ventriculaires délétères ou non**
Aktives implantierbares medizinisches Gerät wie Herzschrittmacher, Defibrillatoren, Kardiovertierer und Multisitevorrichtungen, mit Diskriminierung von schädlichen Extra-Systolen
Implantable medical active device like pacemaker, defibrillator, cardioverter and/or multisite device, with discrimination of deleterious ventricular extrasystoles

(30) Priorité: 04.10.2002 FR 0212292
(43) Date de publication de la demande: 07.04.2004
(73) Titulaire: ELA MEDICAL, 92541 Montrouge (FR)
(72) Inventeur: Casset, Cyril, 75010 Paris (FR)
(74) Mandataire: Dupuis-Latour, Dominique

(56) Documents cités:
- EP-A- 0 550 342
- EP-A- 1 138 346

## Description

L'invention concerne les "dispositifs médicaux implantables actifs" tels que définis par la directive 90/385/CEE du 20 juin 1990 du Conseil des communautés européennes, et plus précisément les dispositifs stimulateurs cardiaques, défibrillateurs et/ou cardioverteurs permettant de délivrer au coeur des impulsions électriques de faible énergie pour le traitement des troubles du rythme cardiaque.

En particulier, elle peut avantageusement concerner les prothèses dites "multisite" dans lesquelles des électrodes sont placées en une pluralité de sites respectifs distincts comportant au moins un site ventriculaire et un site auriculaire. Il peut s'agir d'une prothèse de type "double chambre" (stimulation atriale droite et stimulation ventriculaire droite) ou, le plus souvent, "triple chambre" (stimulation atriale droite et double stimulation ventriculaire) ou "quadruple chambre" (double stimulation atriale et double stimulation ventriculaire).

L'invention concerne les conséquences que peuvent avoir les extrasystoles ventriculaires (ESV) sur le fonctionnement de ces dispositifs.

Une ESV est généralement définie comme une dépolarisation spontanée du ventricule qui n'a pas été précédée, dans un intervalle de temps donné, d'un événement auriculaire associé, spontané (dépolarisation naturelle de l'oreillette) ou stimulé (par une impulsion délivrée par le dispositif à l'oreillette). L'intervalle en question est typiquement un intervalle défini par une fenêtre temporelle de [31; 300 ms] précédant la détection de l'activité ventriculaire.

On peut également considérer qu'il y a ESV lorsque le dispositif détecte une activité ventriculaire précédée d'un événement auriculaire dans un intervalle de temps compris dans la fenêtre [31; 300 ms], mais avec un délai auriculo-ventriculaire (DAV) du cycle cardiaque examiné qui est inférieur d'une durée donnée (par exemple inférieur de plus de 31 ms) au DAV du cycle cardiaque précédent (le cycle cardiaque étant défini comme l'intervalle de temps entre deux événements de même nature dans la même cavité).

Pour de plus amples détails sur les extrasystoles, on pourra se référer au EP-A-0 550 342 (ELA Médical) qui décrit un algorithme de détection et de traitement des ESV. EP-A-1138346 (ELA MEDICAL) pour sa part relate d'un dispositif multisite disposant d'une mesure d'impédance intracardiaque.

En pratique, tous les patients présentent à l'état basal un certain nombre d'ESV isolées, sans conséquence. Mais quand ces extrasystoles deviennent trop fréquentes, le phénomène peut altérer le remplissage des cavités, et donc la fonction hémodynamique du coeur, et/ou favoriser l'apparition de troubles du rythme.

Sur détection d'une ESV, les algorithmes de commande des stimulateurs actuels considèrent qu'il y a présence d'un cycle cardiaque et réinitialisent le comptage des différentes périodes, en particulier de l'intervalle d'échappement ventriculaire (IEV), qui est recyclé afin d'éviter de déclencher une fibrillation ventriculaire par une stimulation à contretemps.

Mais ces dispositifs connus réagissent toujours de manière systématique et indifférenciée lorsqu'ils détectent une ESV.

Le point de départ de l'invention réside dans la constatation du fait que les effets des ESV peuvent être variables, d'un patient à l'autre et également pour un même patient d'une ESV à l'autre, et qu'il peut être opportun de différencier la réaction du dispositif selon les effets prévisibles de l'ESV détectée.

En effet, l'activité ventriculaire spontanée résultant d'une ESV donnée peut avoir des effets physiologiques très variables, allant de l'absence d'effet jusqu'au blocage d'un cycle cardiaque complet, la situation intermédiaire, la plus fréquente, étant une baisse notable du volume sanguin éjecté, du fait d'une contraction de mauvaise qualité ou déphasée par rapport au reste du cycle cardiaque.

Ainsi, par exemple, lorsqu'une ESV survient à la fin de la phase systolique, c'est-à-dire lorsque le myocarde est déjà contracté, l'ESV va bloquer temporairement cette contraction, de sorte qu'il n'y aura plus aucun afflux de sang dans l'organisme pendant la durée de ce blocage. En revanche, si l'ESV survient à un instant où le myocarde n'est pas ou peu contracté, il va provoquer une contraction mécanique de la cavité entraînant l'expulsion d'un volume sanguin.

On comprendra que, s'il on veut optimiser l'hémodynamique du patient, la prochaine stimulation ventriculaire devra intervenir à un instant différent selon le cas, ce qui peut être par exemple obtenu par une adaptation appropriée de l'IEV utilisé par le stimulateur pour définir cet instant (l'IEV est défini comme l'intervalle de temps, compté après une détection ou une stimulation dans le ventricule, à l'issue duquel une stimulation est délivrée à ce même ventricule si aucun événement spontané n'y a été détecté).

Ainsi, l'un des buts de l'invention est, pour chaque ESV détectée, d'opérer une discrimination permettant de déterminer si cette ESV entraîne ou non une contraction mécanique du coeur, notamment pour adapter un paramètre de fonctionnement du dispositif en réaction à cette ESV, par exemple une adaptation de l'intervalle d'échappement utilisé pour définir l'instant de la prochaine stimulation ventriculaire.

Un autre but de l'invention, en variante ou en complément de cette réaction différenciée, est un but statistique, à finalité diagnostique, consistant à comptabiliser par type les ESV détectées - en distinguant entre celles entraînant une contraction mécanique ("ESV non délétères") et les autres ("ESV délétères", avec blocage) -, puis à calculer et mémoriser par exemple la proportion d'ESV délétères par rapport au nombre total d'ESV. Ce paramètre pourra être précieux pour le praticien confronté à l'étude du rythme du patient, en complément de la fréquence d'apparition des ESV ("taux d'extrasystolie"), car il pourra ainsi évaluer non seulement la fréquence des ESV, mais également leur degré global de nocivité pour le patient.

Pour atteindre ce but, l'invention propose, essentiellement, d'enregistrer un signal d'impédance intracardiaque en cas de détection d'ESV, et d'évaluer à partir de ce signal la présence ou non d'une activité mécanique du coeur consécutive à la survenue de l'ESV.

Plus précisément, l'invention propose un dispositif d'un type connu par exemple d'après le EP-A-0 550 342 précité, c'est-à-dire comprenant des moyens de recueil de l'activité cardiaque ventriculaire et auriculaire, et des moyens d'analyse, aptes à détecter dans le rythme cardiaque la survenue d'une extrasystole ventriculaire.

Selon l'invention, ce dispositif comprend en outre : des moyens de mesure, aptes à délivrer un signal d'impédance intracardiaque corrélé au débit sanguin instantané ; et des moyens discriminateurs, aptes à déterminer, en réponse aux variations du signal d'impédance, la présence ou l'absence d'une activité mécanique significative du myocarde consécutive à l'extrasystole ventriculaire détectée par les moyens d'analyse, et pour délivrer en réponse un indicateur du caractère délétère ou non de l'extrasystole ventriculaire en cas d'absence ou, respectivement, de présence de ladite activité mécanique significative du myocarde.

Selon diverses caractéristiques subsidiaires avantageuses :
- le dispositif comprend en outre des moyens aptes à modifier de manière différenciée un paramètre de fonctionnement du dispositif, notamment l'intervalle d'échappement ventriculaire calculé, au cycle suivant la détection de l'extrasystole ventriculaire, en réponse à la détermination, par les moyens discriminateurs, du caractère délétère ou non de l'extrasystole ventriculaire détectée par les moyens d'analyse ;
- les moyens discriminateurs comportent des moyens aptes à évaluer l'accroissement de l'impédance intracardiaque après survenue de l'extrasystole ventriculaire, à comparer cet accroissement à un seuil prédéterminé, et à déterminer la présence d'une activité mécanique significative du myocarde en cas de franchissement de ce seuil ; ce seuil peut notamment être prédéterminé à partir de la valeur de l'impédance mesurée au moment de la survenue de l'extrasystole ventriculaire, augmentée d'un facteur de proportionnalité prédéfini ;
- le dispositif comprend en outre des moyens de comptage, aptes à comptabiliser le nombre respectif d'extrasystoles délétères et non délétères détectées par les moyens d'analyse, et éventuellement à déterminer également les proportions relatives entre extrasystoles délétères et non délétères.

On va maintenant décrire un exemple de mise en oeuvre de l'invention, en référence aux dessins annexés.

La figure 1 est un chronogramme donnant, à côté des marqueurs des événements cardiaques, l'évolution de l'activité électrique du ventricule et celle de la bioimpédance intracardiaque.

La figure 2 est un organigramme explicitant les différentes étapes de la mise en oeuvre de l'invention.

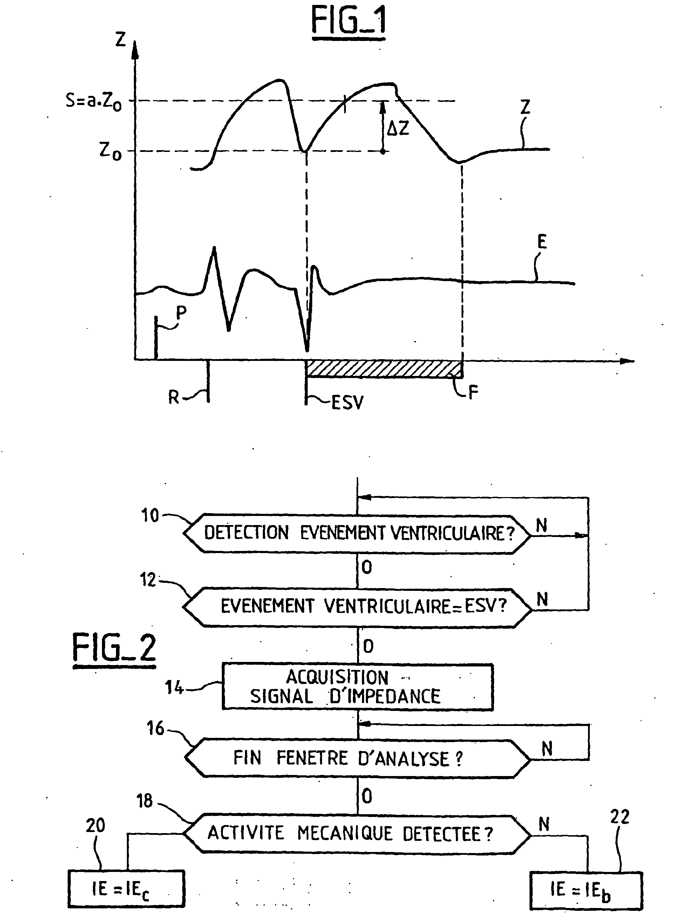

Sur la figure 1, on a illustré en E le tracé électrocardiographique correspondant à un cycle cardiaque normal (défini par le marqueur P de détection d'une activité auriculaire spontanée et le marqueur R de recueil d'une activité ventriculaire spontanée) suivi d'une ESV interrompant ce cycle à un instant qui, dans l'exemple illustré, est assez tardif.

La survenue tardive de cette ESV, au cours donc de la phase diastolique, va entraîner une activité mécanique (contraction) significative du myocarde : il s'agit donc d'une ESV non délétère, et il suffit au dispositif de recycler l'intervalle d'échappement à sa valeur normale, programmée ou calculée.

On entendra par "activité mécanique significative" une activité produisant l'éjection d'un volume sanguin suffisant pour que, du point de vue hémodynamique, le cycle extrasystolique puisse être assimilé à un cycle normal, sans qu'il soit utile de modifier les valeurs des paramètres, programmés ou calculés, de fonctionnement du stimulateur.

En revanche, si cette ESV était survenue beaucoup plus tôt, par exemple en fin de systole, donc au moment où le myocarde était déjà contracté, elle n'aurait pas engendré d'activité mécanique significative supplémentaire et n'aurait eu pour effet que de créer une situation de blocage temporaire. Il pourrait alors être souhaitable non seulement de recycler l'intervalle d'échappement, mais également de modifier la valeur de ce dernier pour une durée plus courte afin de compenser, du point de vue physiologique, la durée de ce blocage.

Pour déterminer la présence ou non d'une activité mécanique significative consécutive à une ESV détectée, le dispositif évalue les variations d'une mesure de bioimpédance intracardiaque, après l'instant de survenue de l'ESV. En effet, les variations de cette bioimpédance sont étroitement corrélées à l'état de contraction du myocarde

On a porté sur la figure 1 les variations de l'impédance intracardiaque Z, dans cet exemple l'impédance transvalvulaire : comme on peut le voir, la caractéristique d'impédance transvalvulaire présente un maximum correspondant à la fin de la systole ventriculaire, moment où le volume sanguin est le plus faible, impliquant donc une valeur d'impédance transvalvulaire élevée.

Pour la mesure de cette impédance, on pourra se référer au EP-A-1 116 497 (ELA médical), qui décrit une technique de mesure de la bioimpédance transvalvulaire (entre oreillette et ventricule situés d'un même côté du coeur) par une configuration tripolaire avec injection d'une impulsion d'un courant de faible amplitude, insuffisant pour exciter les cellules cardiaques, entre deux des sites, et recueil d'un potentiel différentiel entre deux sites différents de cette même configuration.

Le EP-A-1 138 346 (ELA Médical) décrit la mesure d'un autre type de bioimpédance, qui est dans ce cas une bioimpédance transseptale, c'est-à-dire une impédance mesurée entre un site placé d'un côté du coeur (ventricule droit) et un site placé de l'autre côté du coeur (ventricule gauche ou oreillette gauche). Cette technique permet également de délivrer un signal représentatif de l'activité mécanique du coeur, bien que le signal soit plus faible que dans le cas de la mesure d'une bioimpédance transvalvulaire, et soit également influencé par l'impédance propre des tissus du septum.

L'organigramme de la figure 2 illustre les diverses étapes de l'algorithme mis en oeuvre par le dispositif de l'invention.

Tout d'abord (étape 10), le dispositif attend la survenue d'un événement ventriculaire.

Si cet événement ventriculaire est une ESV au sens défini au début de la présente description (étape 12), le dispositif commence alors l'acquisition, par numérisation et échantillonnage, du signal d'impédance intracardiaque Z (étape 14). La première mesure d'impédance intracardiaque, notée Z₀, sert de valeur de référence pour le cycle extrasystolique en cours.

On définit à partir de cette valeur de référence un seuil, par exemple un seuil S = a.Z₀, avec a >1, avantageusement a = 1,5, et l'on poursuit l'acquisition du signal d'impédance pendant la durée d'une fenêtre d'analyse F (étape 16), avantageusement une fenêtre de 150 ms après détection d'une ESV, au cours de laquelle on va rechercher la présence ou non d'une activité mécanique significative du myocarde.

Cette activité mécanique se traduit par une augmentation ΔZ de l'impédance Z, quantité, qui est comparée au seuil S défini comme indiqué plus haut. Le dispositif considérera (étape 18) qu'il y a eu activité mécanique significative si le seuil S a été franchi par le signal Z (d'autres seuils ou méthodes d'évaluation de la variation de l'impédance Z peuvent être bien entendu utilisés, l'important étant de détecter une augmentation notable de la valeur d'impédance par rapport à la mesure initiale Z₀).

Si le seuil est franchi, l'ESV est considérée comme non délétère et l'IEV est maintenu à sa valeur IE_{c} précédemment programmée ou calculée (étape 20), cet IEV étant simplement recyclé à partir de l'instant de survenue de l'ESV. L'intervalle d'échappement déterminé par le dispositif peut être, de manière en elle-même connue, un intervalle d'échappement de base (valeur préprogrammée), ou bien il peut être calculé par une fonction d'asservissement du stimulateur, ou bien encore par des fonctions de lissage ou de prévention, ou une combinaison de ces fonctions.

Si, à l'étape 18, le dispositif détermine que le signal d'impédance n'a pas dépassé le seuil S à la fin de la fenêtre d'analyse F, ceci signifie que le myocarde ne s'est pas ou peu contracté, ce qui sera par exemple le cas si l'ESV est survenue en fin de systole. Cette ESV sans activité mécanique significative consécutive est considérée comme délétère (paramètre qui peut faire l'objet d'un comptage spécifique par le dispositif), et la valeur initiale de l'IEV est modifiée, pour la durée de ce cycle extrasystolique, à une valeur IE_{b} adaptée à la situation de blocage créée par l'ESV.

## Revendications

1. Un dispositif médical implantable actif, notamment un stimulateur cardiaque, défibrillateur et/ou cardioverteur et/ou dispositif multisite, comprenant :
- des moyens de recueil de l'activité cardiaque ventriculaire et auriculaire ; et
- des moyens d'analyse, aptes à détecter dans le rythme cardiaque la survenue d'une extrasystole ventriculaire (ESV) ;
- et des moyens de mesure, aptes à délivrer un signal d'impédance intracardiaque (Z) corrélé au débit sanguin instantané ;
dispositif **caractérisé en ce qu'**il comprend en outre:
- des moyens discriminateurs, aptes à déterminer, en réponse aux variations du signal d'impédance, la présence ou l'absence d'une activité mécanique significative du myocarde consécutive à l'extrasystole ventriculaire détectée par les moyens d'analyse, et pour délivrer en réponse un indicateur du caractère délétère ou non de l'extrasystole ventriculaire en cas d'absence ou, respectivement, de présence de ladite activité mécanique significative du myocarde.

2. Le dispositif de la revendication 1, comprenant en outre :
- des moyens aptes à modifier de manière différenciée un paramètre de fonctionnement du dispositif au cycle suivant la détection de l'extrasystole ventriculaire, en réponse à la détermination, par les moyens discriminateurs, du caractère délétère ou non de l'extrasystole ventriculaire détectée par les moyens d'analyse.

3. Le dispositif de la revendication 2, dans lequel ledit paramètre de fonctionnement modifié de manière différenciée est l'intervalle d'échappement ventriculaire (IE) calculé par le dispositif.

4. Le dispositif de la revendication 1, dans lequel les moyens discriminateurs comportent des moyens aptes à évaluer l'accroissement (ΔZ) de l'impédance intracardiaque (Z) après survenue de l'extrasystole ventriculaire, à comparer cet accroissement à un seuil prédéterminé (S), et à déterminer la présence d'une activité mécanique significative du myocarde en cas de franchissement de ce seuil.

5. Le dispositif de la revendication 4, dans lequel ledit seuil (S) est prédéterminé à partir de la valeur de l'impédance mesurée au moment de la survenue de l'extrasystole ventriculaire, augmentée d'un facteur de proportionnalité (a) prédéfini.

6. Le dispositif de la revendication 1, comprenant en outre :
- des moyens de comptage, aptes à comptabiliser le nombre respectif d'extrasystoles délétères et non délétères détectées par les moyens d'analyse.

7. Le dispositif de la revendication 6, dans lequel les moyens de comptage sont également aptes à déterminer les proportions relatives entre extrasystoles délétères et non délétères.

## Patentansprüche

1. Aktive implantierbare medizinische Vorrichtung, insbesondere ein Herzschrittmacher, Defibrillator und/oder Kardioverter und/oder Multisitevorrichtung, umfassend:
- Mittel zum Empfang der ventrikulären und aurikulären Herzaktivität; und
- Analysemittel, die dazu geeignet sind, im Herzrhythmus das plötzliche Aufheten einer ventrikulären Extrasystole (ESV) zu entdecken;
- und Mittel zu Messung, die dazu geeignet sind, ein Signal der intrakardialen Impedanz (Z) zu liefern, das mit dem momentanen Stromzeitvolumen in Korrelation gebracht wurde;
**wobei die Vorrichtung dadurch gekennzeichnet ist, dass** sie darüber hinaus umfisst:
- Unterscheidungsmittel, die dazu geeignet sind, in Reaktion auf die Schwankungen des Impedanzsignals das Vorhandensein oder die Abwesenheit einer signifikanten mechanischen Aktivität des Myokards festzustellen, die auf die durch die Analysemittel entdeckte ventrikuläre Extrasystole folgt, und um als Reaktion einen Indikator dafür zu liefern, dass der Charakter der ventrikulären Extrasystole schädlich ist oder nicht im Fall der Abwesenheit bzw. des Vorhandenseins der signifikanten mechanischen Aktivität des Myokards.

2. Vorrichtung gemäß Anspruch 1, weiterhin umfassend:
- Mittel, die dazu geeignet sind, auf differenzierte Weise einen Funktionsparameter der Vorrichtung im auf die Entdeckung der ventrikulären Extrasystole folgenden Zyklus zu modifizieren, als Antwort auf die Bestimmung durch die Unterscheidungsmittel, ob der Charakter der durch die Analysemittel entdeckten ventrikulären Extrasystole schädlich ist oder nicht.

3. Vorrichtung gemäß Anspruch 2, in der der auf differenzierte Weise modifizierte Funktionsparameter das durch die Vorrichtung berechnete ventrikuläre Escape-Intervall (IE) ist.

4. Vorrichtung gemäß Anspruch 1, in der die Unterseheidungsmittel Mittel umfassen, die dazu geeignet sind, den Anstieg (ΔZ) der intrakardialen Impedanz (Z) nach dem plötzlichen Auftreten der ventrikulären Extrasystole zu berechnen, diesen Anstieg mit einer vorbestimmten Schwelle (S) zu vergleichen, und die Anwesenheit einer signifikanten mechanischen Aktivität des Myokards im Fall der Überschreitung dieser Schwelle zu bestimmen.

5. Vorrichtung gemäß Anspruch 4, in der die Schwelle (S) ausgehend vom Impedauzwert vorbestimmt ist, der im Zeitpunkt des plötzlichen Auftretens der ventrikulären Extrasystole gemessen wird, erhöht um einen vorbestimmten Proportionalitätsfaktor (a).

6. Vorrichtung gemäß Anspruch 1, weiterhin umfassend:
- Mittel zum Registrieren, die dazu geeignet sind, die jeweilige Zahl von schädlichen und nicht-schädlichen Extrasystolen zu registrieren, die von den Analysemitteln entdeckt werden.

7. Vorrichtung gemäß Anspruch 6, in der die Mittel zum Registrieren auch dazu geeignet sind, die relativen Verhältnisse zwischen schädlichen und nicht-schädlichen Extrasystolen zu bestimmen.

## Claims

1. An active implantable medical device, in particular a cardiac pacemaker, a defibrillator and/or cardioverter and/or multisite device, comprising:
- means for sensing ventricular and atrial cardiac activity;
- analyzing means, adapted for detecting in the heartbeat the occurrence of a ventricular extrasystole (ESV);
- and measuring means, adapted for delivering an intracardiac impedance signal (Z) correlated to the instantaneous blood flow;
said device being **characterised by** further comprising:
- discriminating means, adapted for determining, in response to the variations of the impedance signal, the presence or the absence of a significant mechanical activity of the myocardium consecutive to the ventricular extrasystole detected by the analyzing means, and for issuing in response an indicator of the noxious or non-noxious character of the ventricular extrasystole in case of absence or, respectively, of presence of said significant mechanical activity of the myocardium.

2. The device of claim 1, further comprising:
- means adapted for modifying in a differentiated manner an operating parameter of the device for the cardiac cycle following the detection of the ventricular extrasystole, in response to the determination, by the discriminating means, of the noxious or non-noxious character of the ventricular extrasystole detected by the analyzing means.

3. The device of the claim 2, wherein said operating parameter modified in a differentiated manner is the ventricular escape interval (IE) calculated by the device.

4. The device of claim 1, wherein the discriminating means comprises means adapted for evaluating an increase (ΔZ) in the intracardiac impedance (Z) after the occurrence of the ventricular extrasystole, for comparing said increase to a predetermined threshold (S), and for determining the presence of a significant mechanical activity of the myocardium in case said threshold is crossed.

5. The device of claim 4, wherein said threshold (S) is predetermined from the value of the impedance measured at the time of the occurrence of the ventricular extrasystole, increased by a preset proportionality factor (a).

6. The device of claim 1, further comprising:
- counting means, adapted for counting the respective number of noxious and non-noxious extrasystoles detected by the analysis means.

7. The device of claim 6, wherein said counting means are further adapted for determining the relative proportions between noxious and non-noxious extrasystoles.
